# EUROPEAN PATENT SPECIFICATION

(11) **EP 3 090 727 B1**
(45) Date of publication and mention of the grant of the patent: **24.04.2019**
(21) Application number: 16000072.5
(22) Date of filing: 13.01.2016
(51) Int. Cl.: A61K 8/34, A61Q 7/00, A61K 8/19, A61K 8/23

(54) **HAIR GROWTH COMPOSITION**
HAARWACHSTUMSZUSAMMENSETZUNG
COMPOSITION POUR LA CROISSANCE DES CHEVEUX

(43) Date of publication of application: 09.11.2016
(73) Proprietor: Thiocyn GmbH, 40231 Düsseldorf (DE)
(72) Inventor: Strothmann, Rene, 49186 Bad Iburg (DE)
(74) Representative: f & e patent

(56) References cited:
- EP-A1- 1 287 814
- EP-A2- 0 336 236
- WO-A2-2012/153075
- DE-A1- 19 924 658
- US-A- 4 503 047
- US-A- 5 629 002

## Description

The present invention refers to a composition for preventing and/or treating hair loss and/or for increasing hair growth, comprising ionically bound and/or free thiocyanate ions and a diol compound having 2 to 4 C-atoms, and the use of ionically bound and/or free thiocyanate ions and such a diol for increasing hair growth and/or preventing hair loss in a mammal.

One of the well known development during aging, in particular of men, is the loss of hair and the development of a bald head or bald patch. Driven by esthetical aspects humans are interested in effective compositions improving the hair growth and/or preventing hair loss to reduce the development of a bald head.

Several active agents are known to decrease hair loss / alopecia and to improve hair growth. Some of the most well-known compounds for such a purpose are caffeine and arginine. Each of them are comprised in a great number of hair treatment compositions offered on the market.

Further, thiocyanate is described as being effective in improving hair growth (A. Kramer, W. Weuffen, S. Minnich, S. Koch, M. Minnich, H. Below, B. Thürkow und H. Meffert (1990), Förderung der Haarentwicklung durch Thiocyanat beim Meerschweinchen; Dertamto. Mschr. 176: 417-420). However, thiocyanate applied to the skin doesn't transfer the skin barrier very well, therefore, prior disclosures describe an amount of thiocyanate of at least 1 wt.-% and up to 25 wt.-% of a cosmetic composition for application to the skin (EP 1 287 814 A1).

It was an object of the present invention to provide a composition for preventing and/or treating hair loss and/or for increasing hair growth while decreasing any irritating effect of active agents applied to the skin.

This object is met by a composition for preventing and/or treating hair loss and/or for increasing hair growth comprising:
a) ionically bound and/or free thiocyanate ions in amounts of 0.005 to 1 wt.-% of the total weight of the composition, and
b) a diol compound having 2, 3 or 4 C-atoms, preferably ethanediol, propanediol or butanediol, more preferably ethane 1,2-diol, propane 1,2- or 1,3-diol or butane 1,2-, 1,3- , 2,3- or 1,4-diol and most preferably proyleneglycol (propane- 1,2-diol).

Thiocyanate is also known as rhodanide or - under the IUPAC nomenclature - as cyanosulfanide. The chemical formula of thiocyanate is SCN⁻. According to the present invention the thiocyanate ions are used in form of alkali metal salts, or ammonium salts, preferably the alkali metal salts of thiocyanates are selected from sodium thiocyanate, potassium thiocyanate and/or ammonium thiocyanate, more preferably the alkali metal salts of thiocyanates are selected from sodium thiocyanate and potassium thiocyanate.

The term "ionically bound" refers to the chemical bond that involves electrostatic attraction between oppositely charged ions. According to the present invention the thiocyanate ion SCN⁻ as anion may be ionically bound to any monovalent cation, e.g. a metal cation M⁺ to form a complex of the general formula M⁺SCN⁻. According to the present invention the thiocyanate ions are preferably in form of alkali metal salts or ammonium salts or mixtures of alkali metal salts and ammonium salts, preferably the alkali metal salts of thiocyanates are selected from sodium thiocyanate, potassium thiocyanate and/or ammonium thiocyanate, more preferably the alkali metal salts of thiocyanates are selected from sodium thiocyanate and potassium thiocyanate. On the opposite "free thiocyanate ions" refer to unbound thiocyanate ions.

The diol compound having 2, 3 or 4 C-atoms is preferably one of the group consisting of ethanediol, propanediol or butanediol, more preferably of ethane 1,2-diol, propane 1,2-diol or propane 1,3-diol, butane 1,2-diol , butane 1,3-diol, butane 2,3-diol or butane 1,4-diol and most preferably is proyleneglycol (= propane- 1,2-diol).

The diol compound when applied to the skin of a mammal, increases the transfer rate of the thiocynate (ion) through the skin and supports the entering of the thiocyanate (ion) into hair follicles. By the combination of an thiocyanate (ion) with the diol compound a hair growth composition may comprise an amount of 1 wt.-% or less of a ionically bound and/or free thiocyanate ion to be effective in the treatment or prevention of hair loss or alopecia.

Due to its supporting effect in skin penetration the addition of the diol compound allows the incorporation of thiocyanate (ions) in the composition in a decreased amount compared to products priorly described or marketed, still maintaining the efficiency of this / these compound(s) to stimulate the hair follicle.

The ionically bound and/or free thiocyanate ions are preferably present in amounts of 0.005 to 1 wt.-% of the total weight of the composition, preferably in amounts of 0.005 to less than 1 wt.-%, e.g. in amounts of 0.008 to 0.98 wt.-% of the total weight of the composition, preferred in amounts of 0.01 to 0.95 wt.-% of the total weight of the composition, more preferred in amounts of 0.015 to 0.9 wt.-% of the total weight of the composition, more preferred in amounts of 0.02 to 0.8 wt.-% of the total weight of the composition, more preferred in amounts of 0.025 to 0.7 wt.-% of the total weight of the composition, even more preferred in amounts of 0.03 to 0.6 wt.-% of the total weight of the composition, and most preferred in amounts of 0.03 to 0.5 wt.-% of the total weight of the composition.

The diol compound is preferably used in an amount of 3 to 15 wt.-%, more preferred in an amount of 4 to 12 wt.-%, even more preferred in an amount of 5 to 10 wt.-% and most preferred in an amount of 6 to 8 wt.-% of the total composition.

In a preferred embodiment the thiocyanate (ion) and the diol compound are provided in the composition in a ratio of thiocyanate : diol compound of 1:100 to 1:300, preferably 1:150 to 1:275, more preferably 1:200 to 1:250.

The composition of the present invention may contain further active agents, known as being effective in supporting the decrease of hair loss and/or increasing the hair growth, or agents useful for caring of the skin. Such compounds preferably may be selected from arginine, citric acid, caffeine, allantoin, menthyllactate and panthenol. According to the present invention it is preferred, that at least two of these further ingredients are comprised in the composition, more preferred at least three, even more preferred at least four and most preferred all of the before mentioned ingredients.

Arginine is a basic amino acid known as being effective in the prevention of hair loss and in supporting new hair growth. If arginine is present in the composition, it is preferred that it is comprised in amounts of 0.1 to 5 wt.-% of the total weight of the composition, preferably in amounts of 0.2 to 4 wt.-%, more preferred in amounts of 0.3 to 3.5 wt.-%, even more preferred in amounts of 0.4 to 3 wt.-% and most preferred in amounts of 0.5 to 2.5 wt.-% of the total weight of the composition.

When citric acid is present in the composition, it is preferred that is comprised in amounts of 0.05 to 3 wt.-% of the total weight of the composition, preferably in amounts of 0.1 to 3 wt.-%, more preferred in amounts of 0.2 to 2.5 wt.-% , even more preferred in amounts of 0.25 to 2 wt.-% of the total weight of the composition and most preferred in amounts of 0.3 to 1.5 wt.-% of the total weight of the composition.

It is particularly preferred, that if arginine is comprised in the composition, further citric acid is contained as well. In this case the composition preferably comprises arginine and citric acid in a weight ratio of 5:1 to 1:2, preferably in a weight ratio of 4:1 to 1:1, more preferred in a weight ratio of 3:1 to 1:1 and most preferred in a weight ratio of 2.5:1 to 1.3:1.

By the combination of arginine and citric acid the skin compatibility of the basic amino acid is increased, wherein effectiveness of arginine in preventing hair loss is supported. Thus, in one embodiment of the invention the composition comprises at least thiocyanate (ions), a diol compound as defined above, arginine and citric acid.

In a preferred embodiment of a composition comprising arginine and ionically bound and/or free thiocyanate ions these compounds are comprised in a weight ratio of 300:1 to 5:1, preferably in a weight ratio of 250:1 to 10:1, more preferred in a weight ratio of 200:1 to 12:1 more preferred in a weight ratio of 150:1 to 15:1, and most preferred in a weight ratio of 100:1 to 16:1.

The effect of increasing the skin penetration, provided by the diol compound, indeed refers also to the arginine. Thus, a composition for hair growth comprising at least arginine and a diol component as defined above is also possible.

Allantoin as an active ingredient in cosmetics is known for several beneficial effects, including a moisturizing and keratolytic effect, increasing the water content of the extracellular matrix and enhancing the desquamation of upper layers of dead skin cells, increasing the smoothness of the skin, promoting cell proliferation and wound healing. Therefore, allantoin is commonly used e.g. in cosmetic lotions and creams, shampoos and oral hygiene products. Furthermore, pharmaceutical formulations benefit from its inflammatory-modulating properties and effects on fibroblast proliferation and synthesis of the extracellular matrix.

Allantoin is also known as (2,5-Dioxo-4-imidazolidinyl) urea (IUPAC name), Glyoxyldiureide and 5-Ureidohydantoin.

If comprised in the composition, allantoin is present in amounts of 0.001 to 2 wt.-% of the total weight of the composition, preferably in amounts of 0.005 to 1 wt.-% of the total weight of the composition, more preferred in amounts of 0.01 to 0.5wt.-% of the total weight of the composition, even more preferred in amounts of 0.01 to below 0.1 wt.-%, and most preferred 0.01 to 0.08 wt.-% of the total weight of the composition.

In a preferred embodiment of the invention, the composition comprises at least ionically bound and/or free thiocyanate ions, a diol compound as defined herein above and allantoin.

To obtain a desirable skin caring effect of the present composition allantoin and ionically bound and/or free thiocyanate ions are preferably present in the composition in a weight ratio of 10:1 to 1:40, preferably in a weight ratio of 5:1 to 1:20, more preferred in a weight ratio of 2:1 to 1:10, even more preferred in a weight ratio of 1:1 to 1:8, still more preferred in a weight ratio of 1:1 to 1:6, particularly preferred in a weight ratio of 1:2 to 1:5 and most preferred in a weight ratio of 1:2 to 1:4.

Caffeine as well is well known being effective in prevention of hair loss and in supporting new hair growth. To increase the benefit effect of the thiocyanate (ions) caffeine can be included in the composition of the present invention in an amount of 0.1 to 3 wt.-%, preferably in an amount of 0.25 to 2.5 wt.-%, more preferred in an amount of 0.5 to 2 wt.-%, even more preferred 0. 75 to 1.5 wt.-% and most preferred 0.8 to 1.2 wt.-% of the total composition. In a preferred embodiment caffeine is present in the composition.

The effect of increasing the skin penetration, provided by the diol compound, indeed refers also to the caffeine. Thus, a composition for hair growth comprising at least caffeine and a diol component as defined above is also possible.

Menthyllactate is a compound providing a pleasant and cooling feeling to the skin. If present, said compound can be included in an amount 0.1 to 1 wt.-% of the total composition, preferably in the range of 0.2 to 0.8 wt.-%, more preferred 0.3 to 0.5 wt.-%.

Panthenol is very well known in skin treatment compositions as being effective in alleviate skin irritations, inflammation, dryness or skin defects, e.g. wounds. If panthenol is included in the composition of the present invention, it is preferably present in an amount of 0.1 to 1.5 wt.-%, more preferred 0.3 to 1.2 wt.-%, more preferred 0.5 to 1 wt.-% and most preferred 0.6 to 0.8 wt.-%.

The above mentioned ingredients may be incorporated in any composition comprising further ingredients, e.g. such commonly used for the preparation of cosmetic compositions. Such cosmetic compositions can be represented by e.g. cosmetic solutions, lotions, emulsions, milks, sprays, but further as well by detergent compositions like hair shampoo or conditioner, or other body care products.

The composition of the present invention may be applied to mammalian keratinous tissue, in particular to human or animal skin. The composition may have various forms, for example solutions, suspensions, lotions, creams, gels, sprays, aerosols, cleansing liquid washes, shampoos and hair conditioners, foams, hydrogels, film-forming products, and the like without being limited the mentioned.

Thus, the composition preferably further comprises a dermatologically acceptable carrier wherein the above mentioned components are incorporated to enable the compounds (and optional other ingredients) to be delivered to the skin. The carrier may further comprise any suitable ingredients usually used in according compositions.

The carrier may contain one or more dermatologically acceptable, hydrophilic diluents. Hydrophilic diluents include water, organic hydrophilic diluents such as lower monovalent alcohols (e.g., C1 -C4) and low molecular weight glycols and polyols, including besides the above mentioned diol compond, polyethylene glycol (e.g., molecular weight 200-600 g/mole), polypropylene glycol (e.g., molecular weight 425-2025 g/mole), glycerol, butylene glycol, 1,2,4-butanetriol, sorbitol esters, 1,2,6-hexanetriol, ethanol, isopropanol, sorbitol esters, butanediol, ether propanol, ethoxylated ethers, propoxylated ethers and combinations thereof. Carriers may also be in the form of an emulsion, such as oil-in-water emulsions, water- in-oil emulsions, and water-in- silicone emulsions. An emulsion may generally be classified as having a continuous aqueous phase (e.g., oil-in-water and water-in-oil-in- water) or a continuous oil phase (e.g., water- in-oil and oil-in- water- in-oil). The oil phase may comprise silicone oils, non-silicone oils such as hydrocarbon oils, esters, ethers, and the like, and mixtures thereof. The aqueous phase may comprise water. However, the aqueous phase may also comprise components different from water, including but not limited to water-soluble moisturizing agents, conditioning agents, anti-microbials, humectants and/or other water-soluble skin care actives. Examples of a humectant are glycerin and/or other polyols. Emulsions may also contain an emulsifier. Emulsifiers may be nonionic, anionic or cationic. Particularly preferred as ingredients of the carrier component are water and/or a lower (C1 to C4) alcohol, preferably ethanol and/or isopropanol, mostly preferred ethanol.

A wide variety of optional further components/ingredients may be included in the composition. For example, the composition besides the above particularly mentioned compounds may include absorbents, abrasives, anti-caking agents, antifoaming agents, antimicrobial agents, binders, biological additives, buffering agents, bulking agents, chemical additives, cosmetic biocides, denaturants, cosmetic astringents, drug astringents, external analgesics, film formers, humectants, opacifying agents, fragrances, pigments, colourings, essential oils, skin sensates, emollients, skin soothing agents, skin healing agents, pH adjusters, plasticizers, preservatives, preservative enhancers, propellants, reducing agents, additional skin-conditioning agents, skin penetration enhancing agents, skin protectants, solvents, suspending agents, emulsifiers, thickening agents, solubilizing agents, sunscreens, sunblocks, ultraviolet light absorbers or scattering agents, sunless tanning agents, antioxidants and/or radical scavengers, chelating agents, sequestrants, anti-acne agents, antiinflammatory agents, anti-androgens, depilation agents, desquamation agents/exfoliants, organic hydroxy acids, vitamins and derivatives thereof, and natural extracts. composition of the present invention is free from cortisone or any cortisone derivative.

In particular the following ingredients may be comprises in the compositions of the present invention, wherein dependent from the particular type of composition the suitable ingredients can be selected. E.g. a detergent composition like a shampoo comprises typically any surfactant, water and other suitable ingredients for the carrier, wherein a cream, lotion or milk comprises typically oily and/or fatty components.

All the ingredients suitable for the particular composition type are known to persons skilled in the art. The following examples of ingredients may be comprised in the compositions of the invention:

### Oil/Oil bodies

Suitable oil bodies are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl be- henate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈-alkylhydroxy carboxylic acids with linear or branched C₆-C₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆-C₂₂- fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol^{(R)} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{(R)} TN), linear or branched, symmetrical or asymmetrical dialkyl ethers having 6 to 22 carbon atoms per alkyl group, such as, for example, dicaprylyl ether (Cetiol^{(R)} OE), ring-opening products of epox- idized fatty acid esters with polyols, silicone oils (cyclomethicones, silicone methicone grades, etc.), aliphatic or naphthenic hydrocarbons, such as, for example, squalane, squa- lene or dialkylcyclohexanes, and/or mineral oils.

### Waxes

Among the group of suitable waxes one can differentiate between the following types:
- superfatting agents
- consistency factors
- pearlising waxes, and
- natural waxes

Superfatting agents. Superfatting agents may be selected from substances such as for example, lanolin and lecithin and also polyethoxylated or acylated lanolin and lecithin derivatives, polyol fatty acid esters, monoglycerides and fatty acid alkanolamides; fatty acid alkanolamides can also serve as foam stabilizers.

Consistency factors. The consistency factors can be for example fatty alcohols or hydroxyfatty alcohols containing 12 to 22 and preferably 16 to 18 carbon atoms and also partial glycerides, fatty acids or hydroxyfatty acids of the same carbon range. A combination of these substances with alkyl oligoglucosides and/or fatty acid N-methyl glucamides of the same chain length and/or polyglycerol poly-12-hydroxystea rates is preferably used.

Pearlising waxes. Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxysubstituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

Natural waxes. Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, monta n ester waxes, sasol waxes, hydrogenated jojoba waxes and synthetic waxes such as, for example, polyalkylene waxes and polyethylene glycol waxes.

### Emulsifiers

The emulsifiers, detergents or surfactants may be of non-ionic, anionic, cationic and/or amphoteric nature.

In particular preferred are non-ionic emulsifiers, such as:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- 12/18 fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C₆/₂₂ fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆-₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/16} fatty acid monoesters and diesters of addition products of ethylene oxide onto glycerol are known as lipid layer enhancers for cosmetic formulations. The most preferred emulsifiers are described in more detail as follows:
Partial glycerides. Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

Sorbitan esters. Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

Polyglycerol esters. Typical examples of suitable polyglycerol esters are Polyglyceryl-2 Dipolyhydroxystearate (Dehymuls PGPH), Polyglycerin-3-Diisostearate (Lameform TGI), Polyglyceryl-4 Isostearate (Isolan GI 34), Polyglyceryl-3 Oleate, Diisostearoyl Polyglyceryl-3 Diisostearate (Isolan^{(R)} PDI), Polyglyceryl-3 Methylglucose Distearate (Tego Care 450), Polyglyceryl-3 Beeswax (Cera Bellina), Polyglyceryl-4 Caprate (Polyglycerol Caprate T2010/90), Polyglyceryl-3 Cetyl Ether (Chimexane N L), Polyglyceryl-3 Distearate (Cremophor^{(R)} GS 32) and Polyglyceryl Polyricinoleate (Admul WOL 1403), Polyglyceryl Dimerate Isostearate and mixtures thereof. Examples of other suitable polyolesters are the mono-, di-and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Anionic emulsifiers. Typical anionic emulsifiers are aliphatic C12-22 fatty acids, such as palmitic acid, stearic acid or behenic acid for example, and C12-22 dicarboxylic acids, such as azelaic acid or sebacic acid for example.

Amphotheric or zwitterionic emulsifiers. Other suitable emulsifiers are amphoteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl- 3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of Cocamidopropyl Betaine is particularly preferred. Ampholytic surfactants are also suitable emulsifiers. Am pholytic surfactants are surface-active compounds which, in addition to a C₈/₁₈ alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N- alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C12/18 acyl sarcosine.

### Active principles

The compositions according to the present invention may contain additional ingredients encompassed by the term "active principles". Examples for suitable ingredients are abrasives, anti-acne agents, agents against ageing of the skin, anti-cellulitis agents, antidandruff agents, anti-inflammatory agents, irritation-preventing agents, irritation-inhibiting agents, antioxidants, astringents, perspiration-inhibiting agents, antiseptic agents, anti-statics, binders, buffers, carrier materials, chelating agents, cell stimulants, cleansing agents, care agents, depilatory agents, surface-active substances, deodorizing agents, antiperspirants, softeners, enzymes, essential oils, fibres, film-forming agents, fixatives, foam-forming agents, foam stabilizers, substances for preventing foaming, foam boosters, gelling agents, gel-forming agents, hair care agents, hair-setting agents, hair- straightening agents, moisture-donating agents, moisturizing substances, moisture-retaining substances, bleaching agents, strengthening agents, stain-removing agents, optically brightening agents, impregnating agents, dirt-repellent agents, friction-reducing agents, lubricants, moisturizing agents, opacifying agents, plasticizing agents, covering agents, polish, gloss agents, polymers, powders, proteins, re-oiling agents, abrading agents, silicones, skin-soothing agents, skin-cleansing agents, skin care agents, skin-healing agents, skin-lightening agents, skin-protecting agents, skin-softening agents, hair promotion agents, cooling agents, skin-cooling agents, warming agents, skin-warming agents, stabilizers, UV- absorbing agents, UV filters, detergents, fabric conditioning agents, suspending agents, skin- tanning agents, thickeners, vitamins, oils, waxes, fats, phospholipids, saturated fatty acids, mono- or polyunsaturated fatty acids, hydroxy acids, polyhydroxyfatty acids, liquefiers, dyestuffs, colour-protecting agents, pigments, anti-corrosives, aromas, flavouring substances, odoriferous substances, polyols, surfactants, electrolytes, organic solvents or silicone derivatives and the like as additional auxiliaries and additives.

### Thickening agents and rheology additives

Suitable thickeners are polymeric thickeners, such as Aerosil^{(R)} types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols^{(R)} [Goodrich] or Synthalens^{(R)} [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400^{(R)}, cationic starch, copolymers of diallyl ammonium salts and acrylamides, quaternized vinyl pyrrolidone/vinyl imidazole polymers such as, for example, Luviquat^{(R)} (BASF), condensation products of polyglycols and amines, quaternized collagen polypeptides such as, for example, Lauryldimonium Hydroxypropyl Hydrolyzed Collagen (Lamequat^{(R)} L, Grunau), quaternized wheat polypeptides, polyethyleneimine, cationic silicone polymers such as, for example, amodimethicone, copolymers of adipic acid and dimethylaminohydroxypropyl diethylenetriamine (Cartaretine, Sandoz), copolymers of acrylic acid with dimethyl diallyl ammonium chloride (Merquat 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar CBS, Jaguar C-17, Jaguar C-16 of Celanese, quaternized ammonium salt polymers such as, for example, Mirapol A-15, Mirapol AD-1, Mirapol AZ-1 of Miranol and the various polyquaternium types (for example 6, 7, 32 or 37) which can be found in the market under the tradenames Rheocare CC or Ultragel 300.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Silicones

Suitable silicone compounds are, for example, dimethylpolysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976).

### Sun protection compounds

The compositions according to the invention may contain at least one UV- A filter and/or at least one UV-B filter and/or a broadband filter and/or at least one inorganic pigment.

Preferred cosmetic compositions, preferably topical formulations according to the present invention may comprise one, two, three or more sun protection factors selected from the group consisting of 4-aminobenzoic acid and derivatives, salicylic acid derivatives, ben-zophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid deriva- tives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivatives and indole derivatives.

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L- carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophe- none, uric acid and derivatives thereof, mannose and derivatives thereof, superoxide dismutase, titanium dioxide (for example dispersions in ethanol), zinc and derivatives thereof (for example ZnO, ZnS0₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Advantageous inorganic secondary light protection pigments are finely dispersed metal oxides and metal salts which are also mentioned in WO 2005 123101 A1. Also preferred are particulate UV filters or inorganic pigments, which can optionally be hydrophobed, can be used, such as the oxides of titanium (Ti0₂), zinc (ZnO), iron (Fe₂0₃), zirconium (Zr0₂), silicon (Si0₂), manganese (e.g. MnO), aluminium (Al₂0₃), cerium (e.g. Ce₂0₃) and/or mixtures thereof.

### Anti-ageing agents

Anti-ageing or biogenic agents are, for example antioxidants, matrix-metalloproteinase inhibitors (M M PI), skin moisturizing agents, glycosaminglycan stimulators, anti-inflammatory agents, TRPV1 antagonists and plant extracts.

Antioxidants, amino acids (preferably glycine, histidine, tyrosine, tryptophane) and derivatives thereof, imidazoles (preferably urocanic acid) and derivatives thereof, peptides, preferably D,L-carnosine, D-carnosine, L-carnosine and derivatives thereof (preferably anserine), carnitine, creatine, matrikine peptides (preferably lysyl-threonyl-threonyl-lysyl-serine) and palmitoylated pentapeptides, carotenoids, carotenes (preferably alpha-carotene, beta- carotene, lycopene) and derivatives thereof, lipoic acid and derivatives thereof (preferably dihydrolipoic acid), aurothioglucose, propyl thiouracil and other thiols (preferably thioredoxine, glutathione, cysteine, cystine, cystamine and glycosyl, N-acetyl, methyl, ethyl, propyl, amyl, butyl and lauryl, palmitoyl, oleyl, gamma-linoleyl, cholesteryl, glyceryl and oligoglyceryl esters thereof) and salts thereof, dilauryl thiodipropionate, distearyl thiodipropionate, thiodipropionic acid and derivatives thereof (preferably esters, ethers, peptides, lipids, nucleotides, nucleosides and salts) and sulfoximine compounds (preferably buthionine sulfoximines, homocysteine sulfoximine, buthionine sulfones, penta-, hexa-, heptathionine sulfoximine) in very small tolerated doses (e.g. pmol to micromole/ kg), also (metal) chelators (preferably alpha-hydroxy fatty acids, palmitic acid, phytic acid, lactoferrin, alpha-hydroxy acids (preferably citric acid, lactic acid, malic acid), humic acid, bile acid, bile extracts, tannins, bilirubin, biliverdin, EDTA, EGTA and derivatives thereof), unsaturated fatty acids and derivatives thereof (preferably gamma-linolenic acid, linoleic acid, oleic acid), folic acid and derivatives thereof, ubiquinone and derivatives thereof, ubiquinol and derivatives thereof, vitamin C and derivatives (preferably ascorbyl palmitate, Mg ascorbyl phosphate, ascorbyl acetate, ascorbyl glucoside), tocopherols and derivatives (preferably vitamin E acetate), vitamin A and derivatives (vitamin A palmitate) and coniferyl benzoate of benzoic resin, rutinic acid and derivatives thereof, flavonoids and glycosylated precursors thereof, in particular quercetin and derivatives thereof, preferably alpha-glucosyl rutin, rosmarinic acid, carnosol, carnosolic acid, resveratrol, caffeic acid and derivatives thereof, sinapic acid and derivatives thereof, ferulic acid and derivatives thereof, curcuminoids, chlorogenic acid and derivatives thereof, retinoids, preferably retinyl palmitate, retinol or tretinoin, ursolic acid, levulinic acid, butyl hydroxytoluene, butyl hydroxyanisole, nordihydroguaiac acid, nordihydroguaiaretic acid, trihydroxybutyrophenone, uric acid and derivatives thereof, mannose and derivatives thereof, zinc and derivatives thereof (preferably ZnO, ZnS0₄), selenium and derivatives thereof (preferably selenium methionine), superoxide dismutase, stilbenes and derivatives thereof (preferably stilbene oxide, trans-stilbene oxide) and the derivatives (salts, esters, ethers, sugars, nucleotides, nucleosides, peptides and lipids) of these cited active ingredients which are suitable according to the invention or extracts or fractions of plants having an antioxidant effect, preferably green tea, rooibos, honeybush, grape, rosemary, sage, melissa, thyme, lavender, olive, oats, cocoa, ginkgo, ginseng, liquorice, honeysuckle, sophora, pueraria, pinus, citrus, Phyllanthus emblica or St. John's wort, grape seeds, wheat germ, Phyllanthus emblica, coenzymes, preferably coenzyme Q.10, plastoquinone and menaquinone. Preferred antioxidants are selected from the group consisting of vitamin A and derivatives, vitamin C and derivatives, tocopherol and derivatives, preferably tocopheryl acetate, and ubiquinone.

### Skin-moisturizing agents

Preferred skin moisturizing agents are selected from the group consisting of alkane diols or alkane triols comprising 3 to 12 carbon atoms, preferably C₃-C₁₀-alkane diols and C₃-C₁₀-alkane triols. More preferably the skin moisturizing agents are selected from the group consisting of: glycerol, 1,2-propylene glycol, 1,2-butylene glycol, 1,3-butylene glycol, 1,2-pentanediol, 1,2-hexanediol, 1,2-octanediol and 1,2-decanediol.

### Glycosaminoglycan stimulators

Compositions may comprise substances stimulating the synthesis of glycosaminoglycans selected from the group consisting of hyaluronic acid and derivatives or salts, Subliskin (Sederma, INCI: Sinorhizobium Meliloti Ferment Filtrate, Cetyl Hydroxyethylcellulose, Lecithin), Hyalufix (BASF, I NCI: Water, Butylene Glycol, Alpinia galanga leaf extract, Xanthan Gum, Caprylic/Capric Triglyceride), Stimulhyal (Soliance, I NCI: Calcium ketogluconate), Syn-Glycan (DSM, I NCI: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCI: Water, Butylene Glycol, Phospholipids, Hydrolyzed Sericin), glucosamine, N-acetyl glucosamine, retinoids, preferably retinol and vitamin A, Arctium lappa fruit extract, Eriobotrya japonica extract, Genkwanin, N-Methyl-L-serine, (-)-alpha-bisabolol or synthetic alpha-bisabolol such as e.g. Dragosantol and Dragosantol 100 from Symrise, oat glucan, Echinacea purpurea extract and soy protein hydrolysate. Preferred actives are selected from the group consisting of hyaluronic acid and derivatives or salts, retinol and derivatives, (-)-alpha-bisabolol or synthetic alpha-bisabolol such as e.g. Dragosantol and Dragosantol 100 from Symrise, oat glucan, Echinacea purpurea extract, Sinorhizobium Meliloti Ferment Filtrate, Calcium ketogluconate, Alpinia galanga leaf extract and tetradecyl aminobutyroylvalylaminobutyric urea trifluoroacetate.

### Anti-inflammatory agents

The composition may also comprise anti-inflammatory and/or redness and/or itch ameliorating ingredients, wherein it particularly preferred to use naturally occurring agents. Natural or naturally occurring anti-inflammatory compounds or mixtures of compounds that alleviate reddening and/or itching are in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea; preferably selected from the group consisting of extracts or fractions from camomile, Aloe vera, oats, calendula, arnica, honeysuckle, rosemary, witch hazel, ginger or Echinacea, and/or pure substances, preferably alpha-bisabolol, apigenin, apigenin-7-glucoside, gingerols, shogaols, gingerdiols, dehydrogingerdiones, paradols, natural or naturally occuring avenanthramides, preferably tranilast, avenanthramide A, avenanthramide B, avenanthramide C, non-natural or non-naturally occuring avenanthramides, preferably dihydroavenanthramide D, dihydroavenanthramide E, avenanthramide D, avenanthramide E, avenanthramide F, boswellic acid, phytosterols, glycyrrhizin, glabridin and licochalcone A; preferably selected from the group consisting of alpha-bisabolol, natural avenanthramides, non-natural avenanthramides, preferably dihydroavenanthramide D (as described in WO 2004 047833 A1), phytosterols, glycyrrhizin, and licochalcone A, and/or panthenol, lanolin, (pseudo-)ceramides [preferably Ceramide 2, hydroxypropyl bispalmitamide MEA, cetyloxypropyl glyceryl methoxypropyl myristamide, N-(1- hexadecanoyl)-4-hydroxy-L-proline (1-hexadecyl) ester, hydroxyethyl palmityl oxyhydroxypropyl palmitamide], glycosphingolipids, phytosterols, chitosan, mannose, lactose and beta-glucans, in particular 1,3-1,4- - glucan from oats.

Anti-cellulite agents. Anti-cellulite agents and lipolytic agents are preferably selected from the group consisting of those described in WO 2007/077541, and beta-adrenergic receptor agonists such as synephrine and its derivatives, and cyclohexyl carbamates described in WO 2010/097479. Agents enhancing or boosting the activity of anti-cellulite agents, in particular agents which stimulate and/or depolarise C nerve fibres, are preferably selected from the group consisting of capsaicin and derivatives thereof, vanillyl-nonylamid and derivatives thereof, L-carnitine, coenzym A, isoflavonoides, soy extracts, ananas extract and conjugated linoleic acid.

### Further hair growth activators

The composition according to the present invention may also comprise one or more additional hair growth activators, i.e. agents to stimulate hair growth. Hair growth activators are preferably selected from the group consisting of pyrimidine derivatives such as 2,4- diaminopyrimidine-3-oxide (Aminexil), 2,4-diamino-6-piperidinopyrimidine-3-oxide (Minoxidil) and derivatives thereof, 6-amino-1,2-dihydro-1-hydroxy-2-imino-4-piperidinopyrimidine and its derivatives, further xanthine alkaloids such as theobromine and theophylline and derivatives thereof, quercetin and derivatives, dihydroquercetin (taxifolin) and derivatives, potassium channel openers, antiandrogenic agents, synthetic or natural 5-reductase inhibitors, nicotinic acid esters such as tocopheryl nicotinate, benzyl nicotinate and C₁-C₆ alkyl nicotinate, proteins such as for example the tripeptide Lys-Pro-Val, diphencypren, hormons, finasteride, dutasteride, flutamide, bicalutamide, pregnane derivatives, progesterone and its derivatives, cyproterone acetate, spironolactone and other diuretics, calcineurin inhibitors such as FK506 (Tacrolimus, Fujimycin) and its derivatives, Cyclosporin A and derivatives thereof, zinc and zinc salts, polyphenols, procyanidins, proanthocyanidins, phytosterols such as for example beta-sitosterol, biotin, eugenol, (plus or minus)-beta-citronellol, panthenol, glycogen for example from mussels, extracts from microorganisms, algae, plants and plant parts of for example the genera dandelion (Leontodon or Taraxacum), Orthosiphon, Vitex, Coffea, Paullinia, Theobroma, Asiasarum, Cucurbita or Styphnolobium, Serenoa repens (saw palmetto), Sophora flavescens, Pygeum africanum, Panicum miliaceum, Cimicifuga racemosa, Glycine max, Eugenia caryophyllata, Cotinus coggygria, Hibiscus rosa-sinensis, Camellia sinensis, Ilex paraguariensis, Isochrysis galbana, licorice, extracts of grape, apple, barley or hops or/and hydrolysates from rice or wheat.

### Cooling agents

The compositions may also contain one or more further substances with a physiological cooling effect (cooling agents), which are preferably selected here from the following list: menthol and menthol derivatives (for example L-menthol, D-menthol, racemic menthol, isomenthol, neoisomenthol, neomenthol) menthylethers (for example (1-menthoxy)-1,2-propandiol, (1-menthoxy)-2-methyl-1,2-propandiol, 1-menthyl-methylether), menthylesters (for example menthylformiate, menthylisobutyrate, menthyl-(2-methoxy)acetate, menthyl-(2- methoxyethoxy)acetate, menthylpyroglutamate), menthylcarbonates (for example menthylpropyleneglycolcarbonate, menthylethyleneglycolcarbonate, menthylglycerolcarbonate or mixtures thereof), the semi-esters of menthols with a dicarboxylic acid or derivatives thereof (for example mono-menthylsuccinate, mono-menthylglutarate, mono-menthylmalonate, O-menthyl succinic acid ester-N,N-(dimethyl)amide, O-menthyl succinic acid ester amide), menthanecarboxylic acid amides (in this case preferably menthanecarboxylic acid-N-ethylamide or Na-(menthanecarbonyl)glycinethylester as described in US 4,150,052, menthanecarboxylic acid-N-(4-cyanophenyl)amide or menthanecarboxylic acid-N-(4-cyanomethylphenyl)amide as described in WO 2005 049553 A1, methanecarboxylic acid-N-(alkoxyalkyl)amides), menthone and menthone derivatives (for example L-menthone glycerol ketal), 2,3-dimethyl-2-(2-propyl)-butyric acid derivatives (for example 2,3-dimethyl-2-(2-propyl)-butyric acid-N-methylamide), isopulegol or its esters (1-(-)-isopulegol, I-(-)-isopulegolacetate), menthane derivatives (for example p- menthane-3,8-diol), cubebol or synthetic or natural mixtures, containing cubebol, pyrrolidone derivatives of cycloalkyldione derivatives (for example 3-methyl-2(I-pyrrolidinyl)-2- cyclopentene-I-one) or tetrahydropyrimidine-2-one (for example iciline or related compounds, as described in WO 2004/026840), further carboxamides (for example N-(2- (pyridin-2-yl)ethyl)-3-p-menthanecarboxamide or related compounds), (IR,2S,5R)-N-(4-Methoxyphenyl)-5-methyl-2-(I-isopropyl)cyclohexane-carboxamide, oxamates (preferably those described in EP 2033688 A2).

### Anti-microbial agents

Suitable anti-microbial agents are, in principle, all substances effective against Gram- positive bacteria, such as, for example, 4- hydroxybenzoic acid and its salts and esters, N-(4- chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3- methyl-4-(I-methylethyl)phenol, 2-benzyl-4-chloro-phenol, 3-(4-chlorophenoxy)-I,2- propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GM L), diglycerol monocaprate (DMC), salicylic acid N-alkylamides, such as, for example, n-octylsalicylamide or n-decylsalicylamide.

### Film formers and anti-dandruff agents

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

Suitable antidandruff agents are Pirocton Olamin (1-hydroxy-4-methyl-6-(2,4,4- trimethylpentyl)-2-(1H)-pyridinone monoethanolamine salt), Baypival (Climbazole), Ketoconazol^{(R)} (4-acetyl-I-{4-[2-(2,4-dichlorophenyl) r-2-(1H-imidazol-I-ylmethyl)-1,3-dioxylan-c-4-ylmethoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoeth- anolamide sulfosuccinate Na salt, Lamepon UD (protein/undecylenic acid condensate), zinc pyrithione, aluminium pyrithione and magnesium pyrithione/dipyrithione magnesium sulfate.

### Carriers and Hydrotropes

Preferred cosmetics carrier materials are solid or liquid at 20 °C and 1013 mbar (including highly viscous substances) as for example glycerol, 1,2-propylene glycol, 1,2-butylene glycol, 1,3-propylene glycol, 1,3-butylene glycol, ethanol, water and mixtures of two or more of said liquid carrier materials with water. Optionally, these preparations according to the invention may be produced using preservatives or solubilizers. Other preferred liquid carrier substances, which may be a component of a preparation according to the invention are selected from the group consisting of oils such as vegetable oil, neutral oil and mineral oil.

Preferred solid carrier materials, which may be a component of a composition according to the invention are hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin and inulin and mixtures of two or more of these solids, in particular maltodextrins (preferably with a dextrose equivalent value of 15 - 20), lactose, silicon dioxide and/or glucose.

In addition, hydrotropes, for example further polyols, may be used to improve flow behaviour. Suitable additional polyols besides the diol compound as defined above preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average mo- lecular weight of 100 to 1000 Dalton;
- technical oligoglycerol mixtures with a degree of self-condensation of 1.5 to 10, such as for example technical diglycerol mixtures with a diglycerol content of 40 to 50 percent by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-I,3-diol.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### Perfume oils and fragrances

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert. butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetalde- hyde, cyclamen aldehyde, hydroxycitronellal, lilial and bourgeonal. Examples of suitable ketones are the ionones, isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide N P, evernyl, iraldein gamma, phenylacetic acid, geranyl acetate, benzyl acetate, rose oxide, romillat, irotyl and floramat.

### Detergents/surfactants

Detergents or surfactants usually are present in cosmetic compositions used and suitable for cleaning the skin or body of a mammal. Examples hereof are rinse-off products like shampoo, shower gels, hair conditioner, liquid soap, barred soap or similar.

Suitable detergents are non-ionic surfactants, anionic surfactants, cationic surfactants or amphoteric surfactants. All these type of surfactants suitable for body or hair cleaning are known in the art. Examples for such surfactants are listed below:
Among the numerous cationic surface active agents which may be used are distearyl dimethyl ammonium chloride, stearyl dimethyl benzyl ammonium chloride, stearyl trimethyl ammonium chloride, coco dimethyl benzyl ammonium chloride, dicoco dimethyl ammonium chloride, cetyl pyridinium chloride, cetyl trimethyl ammonium bromide, stearyl amine salts that are soluble in water such as stearyl amine acetate and stearyl amine hydrochloride, stearyl dimethyl amine hydrochloride, distearyl amine hydrochloride, alkyl phenoxyethoxyethyl dimethyl ammonium chloride, decyl pyridinium bromide, pyridinium chloride derivative of the acetyl amino ethyl esters of lauric acid, lauryl trimethyl ammonium chloride, decyl amine acetate, lauryl dimethyl ethyl ammonium chloride, the lactic acid and citric acid and other acid salts of stearyl-1-amido- ethyl-2-heptadecyl-2-imidazoline, quaternaries of such imizadoline with methyl chloride, benzyl chloride, chloroacetic acid and similar compounds, mixtures of the foregoing, and the like. Particularly preferred are stearyl trimethyl ammonium chloride, the lactic or the citric acid salt of stearyl-I-amidoethyl-2-heptadecyl-2-imidazoline, or mixtures thereof.

Any type of anionic surfactant may be used. It is preferable that the anionic surfactant be selected from the group consisting of (C₆-C₃₀) alkyl sulfates, (C₆-C₃₀) alkyl ether sulfates, (C₆-C₃₀) alkylamido ether sulfates, alkylaryl polyether sulfates, monoglyceride sulfates; (C₆-C₃₀) alkylsulfonates, (C₆-C₃₀) alkylamide sulfonates, (C₆-C₃₀) alkylaryl sulfonates, α-olefin sulfonates, paraffin sulfonates; (C₆-C₃₀) alkyl phosphates; (C₆-C₃₀) alkyl sulfoacetates; (C₆-C₂₀)acyl sarcosinates; (C₆-C₃₀) alkylpolyglycoside carboxylic ethers; (C₆-C₃₀) alkylpolyglycoside sulfosuccinates; (C₆-C₃₀) alkyl sulfosuccinamates; (C₆-C₂₄)acyl isethionates; N- (C₆-C₂₄) acyl taurates; C₆-C₃₀ fatty acid salts; coconut oil acid salts or hydrogenated coconut oil acid salts; (C₈-C₂₀)acyl lactylates; (C₆-C₃₀) alkyl-D-galactoside uronic acid salts; and corresponding acids.

The amphoteric surfactants may not be limited specifically. The amphoteric surfactants may be, for example, amine derivatives such as aliphatic secondary or tertiary amine, and optionally quaternized amine derivatives, in which the aliphatic radical is a linear or branched chain comprising 8 to 22 carbon atoms and comprising at least one water-solubilizing anionic group (for example, carboxylate, sulphonate, sulphate, phosphate or phosphonate). The amphoteric surfactant may preferably be selected from the group consisting of betaines and amidoaminecarboxylated derivatives.

The betaine-type amphoteric surfactant is preferably selected from the group consisting of alkylbetaines, alkylamidoalkylbetaines, sulfobetaines, phosphobetaines, and alkylamidoalkylsulfobetaines, in particular, (C₅-C₂₄) alkylbetaines, (C₈-C₂₀) alkylamido (C₁-C₈) alkylbetaines, sulphobetaines, and (C₈-C₂₄) alkylamido (C₁-C₈) alkylsulphobetaines. In one embodiment, the amphoteric surfactants of betaine type are chosen from (C₈-C₂₄) alkylbetaines, (C₅-C₂₄) alkylamido (C₁-C₈) alkylsulphobetaines, sulphobetaines, and phosphobetaines.

Examples of amphoteric surfactant are known under the names cocobetaine, laurylbetaine, cetylbetaine, coco/oleamidopropylbetaine, cocamidopropylbetaine, palmitamidopropylbetaine, stearamidopropylbetaine, cocamidoethylbetaine, cocamidopropylhydroxysultaine, oleamidopropylhydroxysultaine, cocohydroxysultaine, laurylhydroxysultaine, and cocosultaine, and may be used alone or as mixtures.

Among the amidoaminecarboxylated derivatives, mention may be made of the products sold under the name Miranol, as described in U.S. Pat. Nos. 2,528,378 and 2,781,354 and classified in the CTFA dictionary, 3rd edition, 1982 (the disclosures of which are incorporated herein by reference), under the names Amphocarboxyglycinates and Amphocarboxypropionates, with the respective structures:

R1-CONHCH₂CH₂-N⁺ (R2) (R3) (CH₂COO-)

in which:
R1 denotes an alkyl radical of an acid R1-COOH present in hydrolysed coconut oil, a heptyl, nonyl or undecyl radical, R2 denotes a beta-hydroxyethyl group, and
R3 denotes a carboxymethyl group; and

R1'-CONHCH₂CH₂-N (B) (C)

in which:
B represents -CH₂CH₂OX',
C represents -(CH₂)_{Z}-Y', with z=1 or 2,
X' denotes a -CH₂CH₂-COOH group, -CH₂-COOZ', -CH₂CH₂-COOH, -CH₂CH₂-COOZ' or a hydrogen atom,
Y' denotes -COOH, -COOZ', -CH₂-CHOH-S0₃Z' or a -CH₂-CHOH-S0₃H radical,
Z' represents an ion of an alkaline or alkaline earth metal such as sodium, an ammonium ion or an ion derived from an organic amine, and
R1' denotes an alkyl radical of an acid R1'-COOH present in coconut oil or in hydrolysed linseed oil, an alkyl radical, such as a C₇, C₉, C₁₁ or C₁₃ alkyl radical, a C₁₇ alkyl radical and its isoform, or an unsaturated C₁₇ radical.

Preferably, the amphoteric surfactant chosen from amidoaminecarboxylated derivatives may be selected from the group consisting of (C₈-C₂₄) -alkyl amphomonoacetate, (C₈-C₂₄) alkyl amphodiacetate, (C₈-C₂₄) alkyl amphomonopropionates, and (C₈-C₂₄) alkyl amphodipropionates. These compounds are classified in the CTFA dictionary, 5th edition, 1993, under the names Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphopropionate, Disodium Caprylamphodipropionate, Disodium Caprylamphodipropionate, Lauroamphodipropionic acid and Cocoamphodipropionic acid.

The nonionic surfactants themselves are also compounds which are well known per se (in this respect see especially the "Handbook of Surfactants" by M. R. Porter, published by Blackie and Son (Glasgow and London), 1991, pp. 116-178, the disclosure of which is incorporated by reference). Thus, they can, for example, be chosen from alcohols, alpha-diols, alkylphenols and esters of fatty acids that are polyethoxylated, polypropoxylated or polyglycerolated and have at least one fatty chain comprising, for example, from 8 to 18 carbon atoms, it being possible for the number of ethylene oxide or propylene oxide groups to range from 1 to 50, and for the number of glycerol groups to range from 1 to 30. Maltose derivatives may also be mentioned. Mention may also be made of copolymers of ethylene oxide and/or of propylene oxide; condensates of ethylene oxide and/or of propylene oxide with fatty. alcohols; polyethoxylated fatty amides comprising, for example, from 2 to 30 mol of ethylene oxide; polyglycerolated fatty amides comprising, for example, from 1 to 5 glycerol groups, such as from 1.5 to 4; ethoxylated fatty acid esters of sorbitan comprising from 2 to 30 mol of ethylene oxide; ethoxylated oils from plant origin;' fatty acid esters of sucrose; fatty acid esters of polyethylene glycol; fatty acid mono or diesters of glycerol; (C₆-C₂₄) alkylpolyglycosides; N- (C₆-C₂₄) alkylglucamine derivatives, amine oxides such as (C₁₀-C₁₄) alkylamine oxides or N- (C₁₀-C₁₄) acylaminopropylmorpholine oxides; and mixtures thereof.

The nonionic surfactants may be selected from monooxyalkylenated or polyoxyalkylenated, monoglycerolated or polyglycerolated nonionic surfactants. The oxyalkylene units are more particularly oxyethylene or oxypropylene units, or a combination thereof, preferably oxyethylene units.

Examples of oxyalkylenated nonionic surfactants that may be mentioned include: oxyalkylenated (C₈-C₂₄) alkylphenols, saturated or unsaturated, linear or branched, oxyalkylenated C₈-C₃₀ alcohols, saturated or unsaturated, linear or branched, oxyalkylenated C₈-C₃₀ amides, esters of saturated or unsaturated, linear or branched, C₈-C₃₀ acids and of polyethylene glycols, polyoxyalkylenated esters of saturated or unsaturated, linear or branched, C₈-C₃₀ acids and of sorbitol, saturated or unsaturated, oxyalkylenated plant oils, condensates of ethylene oxide and/or of propylene oxide, inter alia, alone or as mixtures.

The surfactants may comprise a number of moles of ethylene oxide and/or of propylene oxide of from 1 to 100 and preferably from 2 to 50. Advantageously, the nonionic surfactants do not comprise any oxypropylene units.

In accordance the present invention, the oxyalkylenated nonionic surfactants are preferably chosen from oxyethylenated C₈-C₃₀ alcohols or ethoxylated fatty esters. It will be noted that alkylpolyglycosides constitute nonionic surfactants which are particularly well suited within the context of the present invention.

Among alkylpolyglucosides which may be used, mention may be made of caprylyl/capryl glucoside, palmkernel/coco glucoside, cetearyl glucoside, decyl glucoside, lauryl glucoside, coco-glucoside, arachidyl gludoside, C₁₂-₂₀ alkyl glucoside, C₁₀-₁₆ alkyl glucoside, myristyl glucoside, myristoyl ethyl glucoside, methyl coco-glucoside, tallowyl ethyl glucoside, undecyl glucoside, octyldodecyl glucoside, isostearyl glucoside, lauroyl ethyl gludoside, cocoyl ethyl glucoside, caproyl ethyl glucoside, and butyl glucoside, but not limited thereto.

All the above mentioned ingredients should be considered as examples for suitable, but optional ingredients of the cosmetic compositions of the present invention. All these additional components are not limiting the invention.

Examples of ingredients preferably used in the present invention may be selected from one or more glyceryl ester and/or one or more polyvalent alcohol, wherein preferably the one or more glyceryl ester is selected from the group consisting of glyceryl stearate and glyceryl caprylate, and preferably the one or more polyvalent alcohol is selected from the group consisting of glycerol and pentylene glycerol.

One further ingredient usable in a cosmetic composition is Laureth-9 (INCI name) also known as Hydroxyl polyethoxy dodecane, Oxypolyethoxydodecane, Dodecylpolyethyleneglycolether, Macrogol lauryl ether or 3,6,9,12,15,18,21,24,27-nonaoxaonatriacontan-1-ol (IUPAC name).

The composition of the present invention can be formulated in any form that is applicable to the body, in particular to the skin or to the hair. For this purpose the compositions of the present invention can be formulated e.g. in form of a liquid composition, a tonic, a solution, a lotion, emulsion, milk, spray or similar, but further as well as a detergent composition like hair shampoo or conditioner, hair gel or styling wax, without being limited to the mentioned.

In particular, the composition of the present invention is for topical use, especially for the treatment of mammal skin, in particular for mammal skin whereon hair growth is desired.

The present invention refers further to the use of ionically bound and/or free thiocyanate ions thereof in combination with a diol compound as defined herein in a ratio of thiocyanate : diol of 1:100 to 1:300 for increasing hair growth or for preventing hair loss in a mammal.

### Examples

### Example 1:

Hair tonic compositions according to the invention were prepared by mixing the following components :

| **Ingredient** | **Amount (kg)** | **Amount (wt.-%)** |
|---|---|---|
| Ethanol 96% , denatured with isopopanol | 350 - 450 | 35 - 45 |
| Propyleneglycol | 50 - 100 | 5-10 |
| Sodium thiocyanate | 0.1 - 0.5 | 0.01 - 0.05 |
| L-Arginine | 0 - 25 | 0.0 - 2.5 |
| Citric acid (monohydrate) | 0 - 12.5 | 0.0 - 1.25 |
| Allantoin | 0.05 - 0.25 | 0.005 - 0.025 |
| Caffein | 0-15 | 0.0 - 1.5 |
| D-panthenol | 5 - 8 | 0.5 - 0.8 |
| Menthyllactate | 2.5 - 5 | 0.25-0.5 |
| Aqua | Ad 1000 | Ad 100 |

### Example 2:

A hair tonic (A) comprising water, ethanol, propyleneglycol, sodium thicyanate, allantoin, panthenol and methyllactate in amounts as shown in Example 1 was prepared by dissolving the components in water / ethanol and mixing the liquid phases.
A hair tonic (B) corresponding to tonic (A), but further comprising caffeine in an amount of 1 wt.-% was prepared.

Both tonics were provided to persons interested in increasing their hair growth due to bald patches, bald heads or hair loss.

A female test person (hair is receding at the temples) used both tonics over a time period of several weeks. She reported about hair growth on the blank areas, wherein said hair was first soft and delicate, but during the time became stronger and thicker. Hair growth was clearly visible after a time period of 8 weeks.

A further female test person suffering from alopecia, having an almost bald head used tonic (A) for a time period of 8 weeks. After this time the head showed a noticeable hair growth (downy hair). In the following the test person used tonic (B) for further 8 weeks, resulting in comfortable hair growth with hair of normal thickness.

All test persons assessed the performance of the tonics as very good, fulfilling their expectation, wherein the caring effect on the scalp as well was positively mentioned.

## Claims

1. A composition for use in a method for preventing and/or treating hair loss and/or for increasing hair growth comprising:
a) ionically bound and/or free thiocyanate ions in amounts of 0.005 to 1 wt.-% of the total weight of the composition, and
b) a diol compound having 2, 3 or 4 C-atoms, preferably ethanediol, propanediol or butanediol, more preferably ethane 1,2-diol, propane 1,2- or 1,3-diol or butane 1,2-, 1,3- , 2,3- or 1,4-diol and most preferably propyleneglycol (propane- 1,2-diol).

2. The composition for use according to claim 1, wherein the thiocyanate ions are in form of alkali metal salts or ammonium salts, wherein preferably the alkali metal salts of thiocyanates are selected from sodium thiocyanate, potassium thiocyanate and/or ammonium thiocyanate, more preferably the alkali metal salts of thiocyanates are selected from sodium thiocyanate and potassium thiocyanate.

3. The composition for use according to any of the preceding claims, wherein ionically bound and/or free thiocyanate ions are present in amounts of 0.005 to less than 1 wt.-%, e.g. in amounts of 0.008 to 0.98 wt.-% of the total weight of the composition, preferred in amounts of 0.01 to 0.95 wt.-% of the total weight of the composition, more preferred in amounts of 0.015 to 0.9 wt.-% of the total weight of the composition, more preferred in amounts of 0.02 to 0.8 wt.-% of the total weight of the composition, more preferred in amounts of 0.025 to 0.7 wt.-% of the total weight of the composition, even more preferred in amounts of 0.03 to 0.6 wt.-% of the total weight of the composition, and most preferred in amounts of 0.03 to 0.5 wt.-% of the total weight of the composition.

4. The composition for use according any of the preceding claims, wherein the composition comprises the diol compound in an amount of 3 to 15 wt.-%, more preferred in an amount of 4 to 12 wt.-%, even more preferred in an amount of 5 to 10 wt.-% and most preferred in an amount of 6 to 8 wt.-%.

5. The composition for use according to any of the preceding claims, wherein the composition further comprises at least one further active ingredient selected from arginine, citric acid, allantoin, caffeine, menthyllactate and panthenol, preferably at least two of them, more preferred at least three of them and most preferred all of the mentioned further ingredients.

6. The composition for use according to any of the preceding claims, wherein arginine is present in amounts of 0.1 to 5 wt.-% of the total weight of the composition, preferably in amounts of 0.2 to 4 wt.-%, more preferred in amounts of 0.3 to 3.5 wt.-%, even more preferred in amounts of 0.4 to 3 wt.-% and most preferred in amounts of 0.5 to 2.5 wt.-% of the total weight of the composition.

7. The composition for use according to any of the preceding claims, wherein citric acid present in amounts of 0.05 to 3 wt.-% of the total weight of the composition, preferably in amounts of 0.1 to 3 wt.-%, more preferred in amounts of 0.2 to 2.5 wt.-% , even more preferred in amounts of 0.25 to 2 wt.-% of the total weight of the composition and most preferred in amounts of 0.3 to 1.5 wt.-% of the total weight of the composition

8. The composition for use according to any of the preceding claims, comprising arginine and citric acid in a weight ratio of 5:1 to 1:2, preferably in a weight ratio of 4:1 to 1:1, more preferred in a weight ratio of 3:1 to 1:1 and most preferred in a weight ratio of 2.5:1 to 1.3:1.

9. The composition for use according to any of the preceding claims, comprising arginine and ionically bound and/or free thiocyanate ions in a weight ratio of 300:1 to 5:1, preferably in a weight ratio of 250:1 to 10:1, more preferred in a weight ratio of 200:1 to 12:1 more preferred in a weight ratio of 150:1 to 15:1, and most preferred in a weight ratio of 100:1 to 16:1.

10. The composition for use according to any of the preceding claims, wherein allantoin is present in amounts of 0.001 to 2 wt.-% of the total weight of the composition, preferably in amounts of 0.005 to 1 wt.-% of the total weight of the composition, more preferred in amounts of 0.01 to 0.5 wt.-% of the total weight of the composition, even more preferred in amounts of 0.01 to below 0.1 wt.-%, and most preferred 0.01 to 0.08 wt.-% of the total weight of the composition.

11. The composition for use according to any of the preceding claims, comprising allantoin and ionically bound and/or free thiocyanate ions in a weight ratio of 10:1 to 1:40, preferably in a weight ratio of 5:1 to 1:20, more preferred in a weight ratio of 2:1 to 1:10 more preferred in a weight ratio of 1:1 to 1:8, more preferred in a weight ratio of 1:1 to 1:6, more preferred in a weight ratio of 1:2 to 1:5 and most preferred in a weight ratio of 1:2 to 1:4.

12. The composition for use according to any of the preceding claims, wherein the composition is an aqueous composition and optionally further comprises one or more further ingredient of a cosmetic composition, selected from oil(s), wax(es), fatty acid(s), detergent(s), surfactant(s), emulsifier(s), and/or one or more alcohol.

13. The composition for use according to ef any of the preceding claims in form of a hair treatment composition, a liquid composition, a tonic, a solution, a lotion, emulsion, milk, spray or similar, hair shampoo or conditioner, hair gel or styling wax.

14. The composition according to any of the preceding claims for use in the treatment of hair loss, thin hair growth, alopecia, bald head or bald patch.

15. Ionically bound and/or free thiocyanate ions in combination with a diol compound as defined in claim 4 in a ratio of thiocyanate: diol compound of 1:100 to 1:300 for use in a method for increasing hair growth or for preventing hair loss in a mammal.

## Patentansprüche

1. Eine Zusammensetzung zur Verwendung in einem Verfahren zur Vorbeugung und/oder Behandlung von Haarausfall und/oder zur Verstärkung von Haarwuchs, enthaltend:
a) ionisch gebundene und/oder freie Thiocyanationen in Mengen von 0,005 bis 1 Gew.-% des Gesamtgewichts der Zusammensetzung, und
b) eine Diolverbindung mit 2, 3 oder 4 C-Atomen, bevorzugt Ethandiol, Propandiol oder Butandiol, weiter bevorzugt Ethan-1,2-diol, Propan-1,2- oder -1,3-diol oder Butan-1,2-, -1,3-, -2,3- oder -1,4-diol und am meisten bevorzugt Propylenglykol (Propan-1,2-diol).

2. Die Zusammensetzung zur Verwendung gemäß Anspruch 1, wobei die Thiocyanationen in Form von Alkalimetallsalzen oder Ammoniumsalzen vorliegen, wobei bevorzugt die Alkalimetallsalze der Thiocyanate ausgewählt sind aus Natriumthiocyanat, Kaliumthiocyanat und/oder Ammoniumthiocyanat, weiter bevorzugt sind die Alkalimetallsalze der Thiocyanate ausgewählt aus Natriumthiocyanat und Kaliumthiocyanat.

3. Die Zusammensetzung zur Verwendung gemäß einem der vorhergehenden Ansprüche, wobei die ionisch gebundenen und/oder freien Thiocyanationen in Mengen von 0,005 bis weniger als 1 Gew.-%, zum Beispiel in Mengen von 0,008 bis 0,98 Gew.-% des Gesamtgewichts der Zusammensetzung vorliegen, bevorzugt in Mengen von 0,01 bis 0,95 Gew.-% der Gesamtmenge der Zusammensetzung, weiter bevorzugt in Mengen von 0,015 bis 0,9 Gew.-% des Gesamtgewichts der Zusammensetzung, weiter bevorzugt in Mengen von 0,02 bis 0,8 Gew.-% der Gesamtmenge der Zusammensetzung, weiter bevorzugt in Mengen von 0,025 bis 0,7 Gew.-% des Gesamtgewichts der Zusammensetzung, noch weiter bevorzugt in Mengen von 0,03 bis 0,6 Gew.-% des Gesamtgewichts der Zusammensetzung und am meisten bevorzugt in Mengen von 0,03 bis 0,5 Gew.-% des Gesamtgewichts der Zusammensetzung.

4. Die Zusammensetzung zur Verwendung gemäß einem der vorhergehenden Ansprüche, wobei die Zusammensetzung die Diolverbindung in einer Menge von 3 bis 15 Gew.-%, weiter bevorzugt in einer Menge von 4 bis 12 Gew.-%, noch weiter bevorzugt in einer Menge von 5 bis 10 Gew.-% und am meisten bevorzugt in einer Menge von 6 bis 8 Gew.-% enthält.

5. Die Zusammensetzung zur Verwendung gemäß einem der vorhergehenden Ansprüche, wobei die Zusammensetzung außerdem wenigstens einen weiteren Inhaltsstoff, ausgewählt aus Arginin, Zitronensäure, Allantoin, Coffein, Menthyllactat und Panthenol, bevorzugt wenigstens zwei von diesen, weiter bevorzugt wenigstens drei von diesen und am meisten bevorzugt alle der genannten weiteren Inhaltsstoffe enthält.

6. Die Zusammensetzung zur Verwendung gemäß einem der vorhergehenden Ansprüche, wobei Arginin in Mengen von 0,1 bis 5 Gew.-% des Gesamtgewichts der Zusammensetzung vorliegt, bevorzugt in Mengen von 0,2 bis 4 Gew.-%, weiter bevorzugt in Mengen von 0,3 bis 3,5 Gew.-%, noch weiter bevorzugt in Mengen von 0,4 bis 3 Gew.-% und am meisten bevorzugt von 0,5 bis 2,5 Gew.-% des Gesamtgewichts der Zusammensetzung.

7. Die Zusammensetzung zur Verwendung gemäß einem der vorhergehenden Ansprüche, wobei Zitronensäure in Mengen von 0,05 bis 3 Gew.-% des Gesamtgewichts der Zusammensetzung vorliegt, bevorzugt in Mengen von 0,1 bis 3 Gew.-%, weiter bevorzugt in Mengen von 0,2 bis 2,5 Gew.-%, noch weiter bevorzugt in Mengen von 0,25 bis 2 Gew.-% des Gesamtgewichts der Zusammensetzung und am meisten bevorzugt in Mengen von 0,3 bis 1,5 Gew.-% des Gesamtgewichts der Zusammensetzung.

8. Die Zusammensetzung zur Verwendung gemäß einem der vorhergehenden Ansprüche, enthaltend Arginin und Zitronensäure in einem Gewichtsverhältnis von 5:1 bis 1:2, bevorzugt in einem Gewichtsverhältnis von 4:1 bis 1:1, weiter bevorzugt in einem Gewichtsverhältnis von 3:1 bis 1:1 und am meisten bevorzugt in einem Gewichtsverhältnis von 2,5:1 bis 1,3:1.

9. Die Zusammensetzung zur Verwendung gemäß einem der vorhergehenden Ansprüche, enthaltend Arginin und ionisch gebundene und/oder freie Thiocyanationen in einem Gewichtsverhältnis von 300:1 bis 5:1, bevorzugt in einem Gewichtsverhältnis von 250:1 bis 10:1, weiter bevorzugt in einem Gewichtsverhältnis von 200:1 bis 12:1, weiter bevorzugt in einem Gewichtsverhältnis von 150:1 bis 15:1 und am meisten bevorzugt in einem Gewichtsverhältnis von 100:1 bis 16:1.

10. Die Zusammensetzung zur Verwendung gemäß einem der vorhergehenden Ansprüche, wobei Allantoin in Mengen von 0,001 bis 2 Gew.-% des Gesamtgewichts der Zusammensetzung vorliegt, bevorzugt in Mengen von 0,005 bis 1 Gew.-% des Gesamtgewichts der Zusammensetzung, weiter bevorzugt in Mengen von 0,01 bis 0,5 Gew.-% des Gesamtgewichts der Zusammensetzung, noch weiter bevorzugt in Mengen von 0,01 bis unterhalb von 0,1 Gew.-% und am meisten bevorzugt von 0,01 bis 0,08 Gew.-% des Gesamtgewichts der Zusammensetzung.

11. Die Zusammensetzung zur Verwendung gemäß einem der vorhergehenden Ansprüche, enthaltend Allantoin und ionisch gebundene und/oder freie Thiocyanationen in einem Gewichtsverhältnis von 10:1 bis 1:40, bevorzugt in einem Gewichtsverhältnis von 5:1 bis 1:20, weiter bevorzugt in einem Gewichtsverhältnis von 2:1 bis 1:10, weiter bevorzugt in einem Gewichtsverhältnis von 1:1 bis 1:8, weiter bevorzugt in einem Gewichtsverhältnis von 1:1 bis 1:6, weiter bevorzugt in einem Gewichtsverhältnis von 1:2 bis 1:5 und am meisten bevorzugt in einem Gewichtsverhältnis von 1:2 bis 1:4.

12. Die Zusammensetzung zur Verwendung gemäß einem der vorhergehenden Ansprüche, wobei die Zusammensetzung eine wässrige Zusammensetzung ist und gegebenenfalls außerdem ein oder mehrere Inhaltsstoff(e) einer kosmetischen Zusammensetzung enthält, ausgewählt aus Öl(en), Wachs(en), Fettsäure(n), Detergenz(ein), Tensid(en), Emulgator(en) und/oder einem oder mehreren Alkoholen.

13. Die Zusammensetzung zur Verwendung gemäß einem der vorhergehenden Ansprüche in Form einer Haarbehandlungszusammensetzung, einer flüssigen Zusammensetzung, eines Tonikums, einer Lösung, einer Lotion, Emulsion, Milch, eines Sprays oder ähnlichem, Haarshampoo oder Conditioner, Haargel oder Stylingwachs.

14. Die Zusammensetzung gemäß einem der vorhergehenden Ansprüche zur Verwendung bei der Behandlung von Haarausfall, dünnem Haarwuchs, Alopezie, Glatze oder Halbglatze.

15. Ionisch gebundene und/oder freie Thiocyanationen in Kombination mit einer Diolverbindung, wie in Anspruch 4 definiert in einem Verhältnis von Thiocyanat : Diolverbindung von 1:100 bis 1:300 zur Verwendung in einem Verfahren zur Verstärkung des Haarwuchses oder zur Vorbeugung von Haarausfall bei einem Säugetier.

## Revendications

1. Composition pour l'utilisation dans un procédé visant à prévenir et/ou traiter une perte de pilosité et/ou stimuler la pousse de cheveux ou de poils, qui comprend :
(a) des ions thiocyanate libres et/ou liés par voie ionique, en des quantités représentant 0,005 % à 1 % du poids total de la composition,
(b) et un composé du type diol comportant 2, 3 ou 4 atomes de carbone, de préférence de l'éthane-diol, du propane-diol ou du butane-diol, mieux encore de l'éthane-1,2-diol, du propane-1,2-diol ou propane-1,3-diol, ou du butane-1,2-diol, butane-1,3-diol, butane-2,3-diol ou butane-1,4-diol, et encore mieux du propylène-glycol (propane-1,2-diol).

2. Composition pour l'utilisation conforme à la revendication 1, dans laquelle les ions thiocyanate se présentent sous la forme de sels de métaux alcalins ou de sels d'ammonium, les sels de métaux alcalins des thiocyanates étant choisis, de préférence, parmi du thiocyanate de sodium, du thiocyanate de potassium et/ou du thiocyanate d'ammonium, et mieux encore les sels de métaux alcalins des thiocyanates étant choisis parmi du thiocyanate de sodium et du thiocyanate de potassium.

3. Composition pour l'utilisation conforme à n'importe laquelle des revendications précédentes, dans laquelle des ions thiocyanate libres et/ou liés par voie ionique se trouvent en des proportions de 0,005 % à moins de 1 % en poids, par exemple en des quantités représentant 0,008 % à 0,98 % du poids total de la composition, de préférence en des quantités représentant 0,01 % à 0,95 % du poids total de la composition, mieux encore en des quantités représentant 0,015 % à 0,9 % du poids total de la composition, encore mieux en des quantités représentant 0,02 % à 0,8 % du poids total de la composition, en particulier en des quantités représentant 0,025 % à 0,7 % du poids total de la composition, encore mieux en des quantités représentant 0,03 % à 0,6 % du poids total de la composition, et au mieux en des quantités représentant 0,03 % à 0,5 % du poids total de la composition.

4. Composition pour l'utilisation conforme à n'importe laquelle des revendications précédentes, la composition comprenant le composé du type diol en une proportion de 3 % à 15 % en poids, mieux encore en une proportion de 4 % à 12 % en poids, encore mieux en une proportion de 5 % à 10 % en poids, et au mieux en une proportion de 6 % à 8 % en poids.

5. Composition pour l'utilisation conforme à n'importe laquelle des revendications précédentes, la composition comprenant en outre au moins un ingrédient actif supplémentaire choisi parmi de l'arginine, de l'acide citrique (nom d'après la nomenclature internationale des ingrédients cosmétiques, INCI : citric acid), de l'allantoïne (nom INCI : allantoin), de la caféine (nom INCI : caffeine), du lactate de menthyle (nom INCI : menthyl lactate) et du dexpanthénol (nom INCI : panthenol), de préférence au moins deux d'entre eux, mieux encore au moins trois d'entre eux, et au mieux tous les ingrédients supplémentaires déjà cités.

6. Composition pour l'utilisation conforme à n'importe laquelle des revendications précédentes, dans laquelle de l'arginine se trouve en des quantités représentant 0,1 % à 5 % du poids total de la composition, de préférence en des proportions de 0,2 % à 4 % en poids, mieux encore en des proportions de 0,3 % à 3,5 % en poids, encore mieux en des proportions de 0,4 % à 3 % en poids, et au mieux en des quantités représentant 0,5 % à 2,5 % du poids total de la composition.

7. Composition pour l'utilisation conforme à n'importe laquelle des revendications précédentes, dans laquelle de l'acide citrique se trouve en des quantités représentant 0,05 % à 3 % du poids total de la composition, de préférence en des proportions de 0,1 % à 3 % en poids, mieux encore en des proportions de 0,2 % à 2,5 % en poids, encore mieux en des quantités représentant 0,25 % à 2 % du poids total de la composition, et au mieux en des quantités représentant 0,3 % à 1,5 % du poids total de la composition.

8. Composition pour l'utilisation conforme à n'importe laquelle des revendications précédentes, qui comprend de l'arginine et de l'acide citrique en un rapport pondéral de 5/1 à 1/2, de préférence en un rapport pondéral de 4/1 à 1/1, mieux encore en un rapport pondéral de 3/1 à 1/1, et au mieux en un rapport pondéral de 2,5/1 à 1,3/1.

9. Composition pour l'utilisation conforme à n'importe laquelle des revendications précédentes, qui comprend de l'arginine et des ions thiocyanate libres et/ou liés par voie ionique en un rapport pondéral de 300/1 à 5/1, de préférence en un rapport pondéral de 250/1 à 10/1, mieux encore en un rapport pondéral de 200/1 à 12/1, encore mieux en un rapport pondéral de 150/1 à 15/1, et au mieux en un rapport pondéral de 100/1 à 16/1.

10. Composition pour l'utilisation conforme à n'importe laquelle des revendications précédentes, dans laquelle de l'allantoïne se trouve en des quantités représentant 0,001 % à 2 % du poids total de la composition, de préférence en des quantités représentant 0,005 % à 1 % du poids total de la composition, mieux encore en des quantités représentant 0,01 % à 0,5 % du poids total de la composition, encore mieux en des proportions de 0,01 % à 0,1 % en poids, et au mieux en des quantités représentant 0,01 % à 0,08 % du poids total de la composition.

11. Composition pour l'utilisation conforme à n'importe laquelle des revendications précédentes, qui comprend de l'allantoïne et des ions thiocyanate libres et/ou liés par voie ionique en un rapport pondéral de 10/1 à 1/40, de préférence en un rapport pondéral de 5/1 à 1/20, mieux encore en un rapport pondéral de 2/1 à 1/10, encore mieux en un rapport pondéral de 1/1 à 1/8, en particulier en un rapport pondéral de 1/1 à 1/6, mieux encore en un rapport pondéral de 1/2 à 1/5, et au mieux en un rapport pondéral de 1/2 à 1/4.

12. Composition pour l'utilisation conforme à n'importe laquelle des revendications précédentes, cette composition étant une composition aqueuse et comportant par ailleurs, en option, un ou plusieurs autre(s) ingrédient(s) de composition cosmétique, choisi(s) parmi un(e) ou des huile(s), cire(s), acide(s) gras, détergent(s), tensioactif(s) et émulsifiant(s), et/ou un ou plusieurs alcool(s).

13. Composition pour l'utilisation conforme à n'importe laquelle des revendications précédentes, sous forme de composition de traitement capillaire, composition liquide, tonifiant, solution, lotion, émulsion, lait, produit à pulvériser ou produit semblable, shampoing ou après-shampoing, gel capillaire ou cire de coiffage.

14. Composition conforme à n'importe laquelle des revendications précédentes, pour l'utilisation dans le traitement d'une chute de cheveux, d'une chevelure clairsemée, d'une alopécie, d'une calvitie ou d'une pelade.

15. Ions thiocyanate libres et/ou liés par voie ionique sous forme de combinaison avec un composé du type diol, répondant à la définition indiquée dans la revendication 4, en un rapport de thiocyanate à composé du type diol valant de 1/100 à 1/300, pour l'utilisation dans un pocédé en vue de stimuler la pousse de cheveux ou de poils, ou prévenir une perte de pilosité chez un mammifère.
